# FASCICULE DE BREVET EUROPEEN

(11) **EP 4 238 554 B1**
(45) Date de publication et mention de la délivrance du brevet: **09.12.2026**
(21) Numéro de dépôt: 23020096.6
(22) Date de dépôt: 28.02.2023
(51) Int. Cl.: A61K 9/00, A61K 47/02

(54) **COMPOSITION ANTIVIRALE À BASE DE SOLUTION D'HYDROXYDE DE CALCIUM**
ANTIVIRALE ZUSAMMENSETZUNG AUF BASIS EINER CALCIUMHYDROXIDLÖSUNG
ANTIVIRAL COMPOSITION BASED ON A CALCIUM HYDROXIDE SOLUTION

(30) Priorité: 03.03.2022 BE 202200016
(43) Date de publication de la demande: 06.09.2023
(73) Titulaire: Dumont, Philippe, 4470 Saint Georges-sur-Meuse (BE)
(72) Inventeur: Dumont, Philippe, 4470 Saint Georges-sur-Meuse (BE)
(74) Mandataire: Powis de Tenbossche, Roland

(56) Documents cités:
- WO-A2-2022/115069
- TR-A2- 202 019 326

## Description

La présente invention a pour objet une composition au moins pour un traitement anti viral au moins au niveau des muqueuses nasales, en particulier d'au moins une partie des voies respiratoires, par exemple des voies respiratoires supérieures. Le traitement de l'invention est à la fois curatif, mais est également préventif. Cette composition permet de protéger et/ou de guérir des personnes infectées par un virus respiratoire ou une bactérie respiratoire, en particulier pour traiter des infections liées à la Covid-19 et à ses variants, ou à des bactéries pneumocoques se développant suite à une affection à la Covid-19.

Des sprays nasaux pour administrer un principe actif via les fosses nasales d'un patient sont connus.

Par le document EP3932412, on connaît une solution nasale aqueuse pour le traitement de la COVID-19. La solution nasale de ce document comprend du Fucoidan, un polysaccharide marin, qui a une activité anti-virale variable sur les virus respiratoires. Le tableau 3 montre que le traitement de contrôle négatif (10 µl de solution saline par pulvérisation dans les fosses nasales) n'est pas efficace, et qu'après deux jours de traitement avec du Fucoidan et un agent antiviral, le nombre de copies du génome viral était non nul, démontrant ainsi une possible contamination entre individu.

Le document US2004/0204399 décrit une composition nasale pour le traitement de sinusite ou de polypes nasales comprenant au moins un agent tensioactif et un agent actif choisi parmi les agents anti-infectieux, les agents anti-inflammatoires, les agents anti-mucolytique, les agents anti-histaminiques, les agents antiseptiques, les agents anti-biotiques, et les combinaisons de tels agents. Parmi les agents antiseptiques, ce document cite à titre d'exemples, l'iode, l'acétate de chlorhexidine, l'hypochlorite de sodium et l'hydroxyde de sodium. Ce document indique que l'hydroxyde de calcium n'est pas le meilleur agent antiseptique pour traiter le Candida albicans en milieu in vitro. Selon les inventeurs de US2004/0204399 (voir paragraphe 0089), il est essentiel d'ajuster la tension superficielle de la solution ou suspension pour atteindre un dépôt effectif de l'agent actif dans les sinus, le surfactant étant utilisé pour assurer un collage des particules au niveau du sinus. Selon ce document la tension superficielle est dès lors de préférence comprise entre 30 et 40 dynes/cm (entre 30 et 40 millinewton/m), soit une tension superficielle inférieure à la tension superficielle de l'eau de par la présence d'une quantité importante de surfactant. Le pH de la composition est ajusté à des valeurs comprise entre 3 et 8,5.

Le document CA2715140 décrit une méthode pour générer des radicaux hydroxyles, radicaux considérés comme efficaces pour inactiver des virus. Cette génération de radicaux est obtenue par la mise en contact par la mise en contact une poudre d'un oxyde d'un ou plusieurs des métaux choisis parmi les les métaux alcalins, les métaux alcalino-terreux, les métaux des groupes 4 à 12 du tableau périodique et l'aluminium, avec un ou plusieurs hydroxydes choisis parmi les hydroxydes de métal alcalin, les hydroxydes de métal alcalino-terreux, l'hydroxyde de fer, l'hydroxyde de cuivre, l'hydroxyde de zinc, l'hydroxyde d'aluminium et l'hydroxyde d'ammonium. Ce document n'enseigne pas une solution aqueuse pour application nasale.

Le document "Factors affecting stability and infectivity of SARS-CoV-2"; Chan et al, Journal of Hospital Infection, 106, 2020, pages 226-231 décrit la viabilité du virus dans des environnements différents. Le milieu viral a été traité à des pH différents avec de l'acide chlorhydrique et une solution de soude caustique. le tableau II montre que le traitement avec de l'acide chlorhydrique en une quantité suffisante pour abaisser le pH à moins de 4 ou avec de la soude caustique pour accroître le pH à plus 10 permet une réduction de la charge virale 6 jours après le début du traitement. Ce document montre que les désinfectants domestiques ont un effet rapide sur la réduction de la charge virale. Parmi les désinfectants listés, le "dermo docyn" est cité, cette solution est à base d'eau, d'acide hypochlorite et d'hypochlorite de sodium, c'est-à-dire à base d'eau de javel. Ce document ne fait nullement référence à un traitement nasal.

Selon certains auteurs (Cold exposure impairs extracellular vesicle swarm-mediated nasal antiviral immunity", Di Huang et al, Journal Allergy Clin Immunology, 2022 American Academy of Allergy, Asthma & Immunology, accepté pour publicatation le 20 septembre 2022), l'immunité antivirale nasale serait dépendante de la sécrétion de vésicules extracellulaires provenant de l'épithélium des fosses nasales. Selon ces auteurs, avec un environnement froid (4°C), le nombre de vésicules extracellulaires libérés dans les fosses nasales est réduit par rapport à des températures de 23°C, ce qui pourrait expliquer les variations saisonnières des infections respiratoires.

Le document TR202019326 et son équivalent WO2022/115069 décrivent une solution nasale hypertonique présentant un pH de valeur 7 ou plus comprenant sous forme soluble en tant qu'agents tampons le carbonate de sodium et/ou le bicarbonate de sodium et/ou le carbonate de potassium et/ou le bicarbonate de potassium et/ou le tripolyphosphate de sodium, etc. Tel qu'indiqué en page 6, lignes 11 à 20 du document WO2022/115069, la solution nasale hypertonique comprend de préférence du carbonate et/ou du bicarbonate, du sodium pour assurer un effet de soulagement et/ou du potassium pour réparer les tissus épithéliales des voies respiratoires.

Sans être lié à une quelconque théorie, il semble que l'efficacité du traitement contre les virus à partir d'un spray selon l'invention est due au moins par une neutralisation des sucres au niveau des fosses nasales et des voies respiratoires supérieures, un effet de recouvrement formant un film barrière contre les attaques virales, un effet à long terme (la réaction de carbonatation de la chaux hydratée en carbonate de calcium est lente), un effet tampon maintenant un pH élevé en surface du dépôt sur les fosses nasales, dépôt basique ayant une forte affinité pour les virus et autres ARN. Cette meilleure efficacité pourrait être due par une certaine atténuation du virus présent dans le nez ou les voies supérieures respiratoires, ce virus atténué étant alors dans une forme plus apte à être attaqué par les défenses naturelles de l'organisme, ledit organisme formant ainsi plus rapidement des anticorps pour se défendre contre les effets d'un ou de virus et/ou d'une ou de bactéries. La composition selon l'invention agirait dans les voies nasales en une sorte de vaccin à virus atténué ou à bactérie atténué, ce vaccin étant à large spectre d'activité. Ceci est particulièrement intéressant, car souvent un organisme est affaibli par l'attaque d'un virus, avant d'être attaqué par une bactérie. ("la majorité des décès survenus lors de la grippe espagnole avait pour origine des surinfections bactériennes, notamment due au pneumocoque." en page 529 de l'article "La grippe et les surinfections bactériennes, Menaces et traitements", Roquilly et al, M/S Médecine/Sciences, 2017, 33, pages 528 à 533)

L'invention a pour objet d'offrir à tous les pays du Monde, et donc à la population mondiale, un traitement rapide, efficace, simple à utiliser, facile à stocker, sans effet secondaire, stable dans le temps, et peu coûteux pour lutter contre la pandémie de la Covid-19 et de ses variants, ainsi que pour lutter contre des attaques virales futures.

L'invention a encore pour objet un procédé de fabrication ou de préparation d'une composition antivirale selon l'invention, ce procédé comprenant une mise en solution aqueuse d'une molécule non irritante, non toxique et sans effet secondaire pour les cellules humaines des voies respiratoires, mais qui a un effet biocide contre les micro-organismes pathogènes, en particulier les coronavirus, tels que les coronavirus respiratoires, par exemple du type Covid-19, mais également contre des bactéries et autres parasites.

L'invention a aussi pour objet d'apporter un moyen de traitement supplémentaire aux moyens de traitement actuels, moyens thérapeutiques actuels, en particulier les vaccins, pour lutter contre la pandémie actuelle de la Covid-19 et de ses variants. La composition de l'invention permettra alors d'éradiquer plus facilement les épidémies futures, et d'éviter une propagation foudroyante au niveau mondial de virus et bactéries, suite aux échanges internationaux fréquents et rapides.

La composition selon l'invention est utile pour booster et/ou améliorer la défense immunitaire des personnes, en particulier à titre préventif, mais également à titre curatif. La composition selon l'invention a un effet de potentialisation des traitements anti-viraux existants, de l'effet des vaccins, en particulier de l'effet des vaccins à ARN messager. Sans être lié à une quelconque théorie, l'invention permet de couvrir les parois des fosses nasales et les poils de celles-ci, de manière favoriser la sécrétion de vésicules extra-cellulaires pour booster l'immunité naturelle antivirale au niveau du nez ou des voies respiratoires supérieures.

La composition selon l'invention est donc un outil utile dans la lutte contre la Covid-19 permettant de réduire le nombre d'hospitalisations, le nombre de placements de patients en réanimation, avec ventilation assistée, de réduire le nombre de décès, et dès lors de réduire les surcharges de travail pour le personnel en milieu hospitalier.

Par rapport aux vaccins développés pour la Covid-19, leur utilisation à large échelle a montré les limites suivantes de ceux-ci:
- la protection contre la Covid-19 n'est que partielle entre 50 et 90%, et variable car dépendant des mutations du virus. Contrairement aux vaccins, la chaux hydratée est active contre tous virus et mutants de ceux-ci.
- une diminution de leur efficacité dans le temps, nécessitant des rappels de vaccination tous les 4 à 6 mois, ces vaccinations provoquant chez certains patients, des douleurs dans les 10 à 15 jours qui suivent l'injection opérée par du personnels hospitaliers habilités et qualifiés. L'hydroxyde de calcium peut être administré dans les narines d'un patient (par le patient lui-même) une ou deux ou trois fois par jour, pendant une semaine. Cette application de microdoses d'hydroxyde de calcium dans les voies nasales se fait sans aucune gène et ne nécessite pas l'intervention d'un médecin pour son administration. Cette administration peut se faire au moyen d'un simple spray nasal. Vu la propriété antivirale de la solution, l'utilisation d'un spray d'un patient pour administrer une dose chez un autre patient n'entraîne pas de propagation du virus entre les patients.
- le vaccin est développé de manière spécifique pour un virus, ce virus étant à même de muter pour échapper à l'effet du vaccin. L'hydroxyde de calcium est efficace dans le traitement du virus originel, de ses protéines virales, de certains mutants de celui-ci, etc. L'hydroxyde de calcium déposé dans les fosses nasales et sur les poils du nez présente une grande surface spécifique et présente une action importante. L'action de l'hydroxyde de calcium permet d'agir simultanément sur des virus et des bactéries , permettant ainsi de booster l'immunité naturelle d'un patient soumis à la fois à une attaque virale et à une attaque bactérienne.
- le vaccin ARN a démontré une inefficacité à éviter la transmission de la covid-19 ou de protéine virale de la Covid-19. Les particules ou gouttelettes sortant du nez d'une personne infectée de la Covid-19 sont encore chargées d'une charge virale Covid importante. Lors d'un traitement à l'hydroxyde de calcium dans les narines, les virus ou protéines virales expulsés par le nez sont traités et rendus au moins partiellement inertes et/ou atténués. Ceci est particulièrement intéressant, car cela permet d'éviter qu'un masque ou un mouchoir ne devienne un milieu de prolifération du virus ou de mutants expulsés par le nez d'une personne infectée. De plus en cas de changement de masque, le masque usager présente une moindre charge virale, ce qui permet d'éviter des risques de contaminations pour le personnel chargé de l'entretien et du nettoyage de locaux.
- les vaccins sont souvent difficiles à produire, en des quantités suffisantes pour atteindre une immunité globale immédiate. Devant l'urgence d'une maladie, les procédures normales de délivrance des autorisations de mise sur le marché ne sont pas suivies. Des agents de stabilisation ou de protection des virus atténués ou des protéines ARN sont nécessaires. Ces vaccins doivent être conservés dans des frigos, parfois à très basse température. La fabrication de sprays nasaux contenant de l'hydroxyde de calcium dissous, est aisée, car l'hydroxyde de calcium dissous est stable, car l'hydroxyde de calcium dissous est un agent antiviral assurant la conservation de la solution à pulvériser, et car l'hydroxyde de calcium dissous est un agent de protection contre la prolifération de virus ou bactéries sur les parois intérieure et extérieure du contenant ou du spray ou de la recharge.
- Les vaccins ARN font l'objet de critiques d'une partie de la population, celle-ci ayant des craintes et une méfiance quant à des effets secondaires potentiels à long terme liés à ceux-ci. La composition selon l'invention ne présente aucun effet secondaire.
- la pandémie est équivalente à un feu de forêt. Il est essentiel de l'éteindre rapidement. La mondialisation et le transport aérien sont des vecteurs accélérant la transmission de la maladie. La mise en place d'une infrastructure de vaccination et le développement d'un nouveau vaccin requièrent du temps et du personnels qualifiés, pour la production, la logistique et la vaccination proprement dite. Le spray nasal selon l'invention est de fabrication simple, tandis que son utilisation peut être opérée par le patient lui-même. Si la fabrication des vaccins est opérée dans un nombre restreint de sites, envoyant alors les vaccins préparés dans le monde entier, la fabrication du spray selon l'invention peut être opérée dans tous laboratoires comprenant une chambre blanche, à partir de chaux hydratée sèche (en particulier de grande surface spécifique, par exemple de la chaux hydratée avec une surface spécifique BET de plus de 20m²/g, telle que 25m²/g, 30m²/g, 35 m²/g, 40m²/g, voire plus). Le transport d'un sac de chaux hydratée sèche vers un laboratoire de fabrication peut se faire par un véhicule ou par avion sans risque de perte d'efficacité et sans danger pour la population, un tel transport permettant en plus d'éviter de transporter un grand volume d'eau. (si la composition comprend 1g de chaux hydratée par litre, la préparation des sprays dans un lieu proche de son lieu d'utilisation à partir de 25kg de chaux hydratée sèche permet d'éviter le transport de 25 tonnes d'eau.)

L'invention concerne une composition liquide de traitement antiviral et/ou antibactérien et/ou anti inflammatoire et/ou décongestionnant au moins au niveau de la muqueuse nasale d'un mammifère et/ou pour booster la défense immunitaire d'un mammifère, ladite composition liquide se présentant sous la forme d'une solution aqueuse à mettre en contact avec la muqueuse nasale, ladite solution aqueuse liquide étant constituée à plus de 99,5% en poids d'eau déminéralisée et d'hydroxyde de calcium et comprenant de l'hydroxyde de calcium dissous avec une teneur en hydroxyde de calcium dissous comprise entre 0,3g/litre et 3g/litre, le pH de la solution étant supérieur à 11,5 à 20°C, tandis que la solution aqueuse présente une tension superficielle à 20°C supérieure à 0,06 N.m⁻¹ (60 dynes/cm), de préférence égale ou supérieure à environ 0,07 N.m-1 (70 dynes/cm), ladite composition étant exempte de particules solides d'hydroxyde de calcium, et autres particules solides.

La composition de l'invention permet de disposer d'un traitement pour éradiquer rapidement un épidémie d'un virus, dès son émergence. La composition permet un traitement sans effet secondaire néfaste, un traitement efficace contre le virus originel, mais également contre ses mutants, un traitement simple et accessible à tous, un traitement bon marché, sans effet secondaire. Le spray nasal peut facilement être reconditionné, par un simple remplissage du spray avec une quantité de solution antivirale active, la solution antivirale étant apte pour traiter les parois internes du contenant, garantissant ainsi l'absence de virus dans le contenant, même si ce dernier n'a pas été soumis à une étape de traitement thermique.

Un objet de l'invention est de répondre à ces impératifs. Elle propose un procédé de traitement de virus, en particulier de coronavirus, plus spécifiquement de la covid - 19 et de ses variants, par utilisation d'une molécule inorganique peu soluble dans l'eau, à savoir l'hydroxyde de calcium Ca(OH)₂ en solution aqueuse. Sans rentrer dans les détails, la présence d'ions hydroxyles dans la solution est responsable de propriétés clés d'une activité virale et antimicrobienne à large spectre, d'une pénétration dans les biofilms, d'une inhibition d'endotoxines et d'une dissolution des tissus organiques (Athanassiadis et al., "The use of calcium hydroxide, antibiotics and biocides as antimicrobial medicaments in endodontics", Australian Dent. Journal, 2007, 52 (Supplément S1), pages 64 à 82). L'effet létal des ions hydroxyles est obtenu par divers mécanismes, dont une attaque des membranes cytoplasmiques microbiennes, une attaque de l'ADN, une inhibition de la réplication d'ADN, et une suppression d'activité enzymatique. L'hydroxyde de calcium tue des micro-organismes, des champignons, et empêche leur croissance. ( Orstavik et al, "Root canal disinfection: A review of concepts and recent developments", Aust. Endod. Journal 2003, 29, pages 70 à 74).

Le traitement consiste à appliquer sur les muqueuses nasales et/ou sur les poils du nez et/ou dans une partie des voies respiratoires, en particulier des voies respiratoires supérieures, une quantité d'hydroxyde de calcium dissous. Cette application peut se faire par inhalation ou nébulisation d'une solution aqueuse d'hydroxyde de calcium. La solution d'hydroxyde de calcium présente une concentration en ions hydroxyles pour atteindre un pH optimal, en particulier de 11,5 à 12,5. Cette application se fait sans risque d'obturation des ouvertures du dispositif lors du passage du soluté. Cet hydroxyde de calcium se déposé alors sous forme d'une fine couche ou pellicule sur des parois des fosses nasales, et/ou des poils du nez et/ou des parties supérieures des voies respiratoires.

On utilise de préférence un spray nasal pour des injections de doses unitaires fréquentes, à titre curatif et préventif. A titre curatif, par exemple dès l'apparition de premiers symptômes (toux, fièvre, agueusie, anosmie, grande fatigue, etc.), on utilise avantageusement un nébuliseur ou un aérosol électrique pour administrer dans une partie des voies respiratoires une ou des doses successives d'hydroxyde de calcium.

L'hydroxyde de calcium est la première molécule de synthèse connue par l'homme. Pour protéger son feu, l'homosapiens l'entourait avec des pierres calcaire, matériau très abondant. La chaleur dégagée par la combustion du bois transformait le carbonate de calcium en oxyde de calcium (étape de calcination des pierres calcaire). Cette réaction chimique (calcination) était aisément visible par l'homosapiens, puisque lors de pluie, l'oxyde de calcium se transformait en hydroxyde de calcium accompagné par un délitement et un dégagement important de chaleur. Ces phénomènes n'ont pas pu leur échapper.

Une première utilisation de cet hydroxyde de calcium a été le blanchiment, comme on a pu le constater par des traces sur des parois laissées par des peuplades primitives.

L'hydroxyde de calcium a déjà sauvé l'humanité de la peste et du choléra. Ainsi, la ville de Bruges a été sauvée de la peste en 1436, par des mesures de confinement et le port d'un masque un nez allongé chargé de laine imbibée de chaux hydratée, cette laine formant un filtre germicide.

Paracelse (1493-1541), médecin suisse, botaniste, alchimiste préconisait de badigeonner les murs et les rues de Salzbourg au lait de chaux, afin de détruire le bacille de la peste. En 1866, la commune de Cortil-Wodon (Belgique) sujette à une épidémie de peste invitait ses habitants à blanchir l'intérieur de leurs habitations et fournissait gratuitement la chaux aux indigents.

L'usage de la chaux est tombée en désuétude par l'arrivée sur le marché de désinfectants modernes complexes, et par l'absence de recherche fondamentale par l'industrie chaufournière.

Il faut noter que l'action germicide de l'hydroxyde de calcium est encore utilisée actuellement pour traiter la diarrhée des nourrissons, en ajoutant de l'hydroxyde de calcium au lait en poudre, avant transformation de cette poudre en un lait liquide. Une telle utilisation actuelle démontre l'innocuité de la molécule.

En 1974, une prise de conscience de l'impérieuse nécessité de recherche et développement dans le domaine de la chaux a permis à l'industrie chaufournière de mettre au point un hydroxyde de calcium de très grande réactivité. Cette chaux en poudre de grande réactivité est par exemple décrite dans les brevets US5,173,279 et US 5,277,837. Cette technologie a été utilisée dans de nombreux sites de production.

Ces résultats reposent sur une recherche fondamentale et sur la compréhension de la réaction d'extinction de la chaux vivre, réaction qui paraît simple, mais qui est en réalité complexe, puisqu'elle implique une phase de dissolution d'oxyde de calcium, une cristallisation de l'hydroxyde de calcium, et une combinaison de réactions endothermiques et exothermiques. Ces recherches ont abouti à la préparation d'une molécule 'hydroxyde de calcium de très grande réactivité utile pour des usages divers, dont entre autres pour le traitement de fumées et pour leur action germicide contre les pathogènes.

Dans le cadre d'une étude sur la dangerosité et la toxicité des produits chimiques pour l'Union Européenne, de nombreux spécialistes de la santé (ophtalmologues, pneumologues, dermatologues) ont été contactés pour connaître la présence d'effets secondaires indésirables ou non de la chaux hydratée. Dans le cadre de cette étude, plusieurs médecins du travail ont fait part de leur constatation que tous les travailleurs travaillant au niveau d'installations d'hydratation et de séchage de l'hydroxyde de calcium ne développaient pas de problème pulmonaire, ni de tuberculose, en dépit d'une atmosphère riche en fines particules d'hydroxyde de calcium . Lorsque les normes de protection des travailleurs n'étaient pas très sévères, la quantité importante de fines particules de chaux hydratée en suspension provoquait des toux et certains saignements au niveau du nez de certains ouvriers, mais sans réel effet sur leur santé. Ces constatations corroborent l'innocuité des micelles de la chaux, micelles aussi appelées "fleur de chaux". Dans le livre de Boero J., "Fabrication et emploi des chaux hydrauliques", 1925, Librairie Polytechnique, Béranger, Liège, l'ingénieur Boero décrit une méthode d'extinction de chaux, aujourd'hui désuète. Dans cette méthode, la chaux vive en roches était déversée dans des fosses et aspergée d'eau, ce qui provoquait un dégagement intense de chaleur et de poussières. Lorsque la chaux était considérée comme suffisamment éteinte,les portes des salles d'extinction étaient ouvertes et des ouvriers reprenaient la chaux hydratée au moyen de pelles et de brouettes. Cette reprise de la chaux hydratée hors des fosses était un travail pénible. Malgré l'atmosphère riche en poussières de chaux hydratée, l'ingénieur Boero stipule en page 68 de son livre : "Nous devons dire que les poussières de chaux ne paraissent pas avoir une action mauvaise sur l'organisme. Nous avons eu des équipes d'ouvriers qui après quinze ans de travail n'étaient nullement atteints dans les voies respiratoires."

Ces observations sont essentielles car elles mettent en évidence l'innocuité de l'hydroxyde de calcium. Elles ont été corroborées sur un grand nombre de volontaires qui ont accepté le traitement sur base des connaissances acquises, dans des conditions beaucoup plus sévères qu'une simple inhalation de solution d'eau de chaux par un spray avec des doses unitaires de 50 à 200µl.

Lors des tests décrits dans les exemples, aucun saignement au niveau du nez, aucune toux allergique , ni même aucune gêne ou autre désagrément n'ont été observés.

L'utilisation de la composition de l'invention est basée sur deux notions essentielles, à savoir d'une part, l'innocuité et l'absence d'effets secondaires de l'hydroxyde de calcium, et d'autre part, son action virucide à très large spectre.

L'utilisation repose aussi sur une connaissance approfondie de certaines propriétés remarquables et spécifiques de la molécule d'hydroxyde de calcium, molécule choisie de manière spécifique parmi toutes les molécules inorganiques, vu des propriétés remarquables de l'hydroxyde de calcium.

Une première propriété remarquable de l'hydroxyde de calcium en milieu aqueux est la formation d'une solution tampon avec un pH stabilisé variant entre 11,449 à 60°C et 13,423 à 0°C. Une telle solution aqueuse présente donc un pH alcalin suffisant pour détruire les virus et autres micro-organismes, sans affecter les cellules ADN du corps humain. Ce qui est spécialement remarquable ici est que lorsque la température de traitement est réduite, le pH de la solution devient plus alcalin (pH plus agressif), tandis que lorsque la température de la solution est augmentée (température ayant un effet virucide), le pH est réduit tout en restant toujours suffisamment alcalin pour avoir une action antivirale. Le traitement antiviral de l'hydroxyde de calcium contre des virus non désirés est donc contrôlé par le couple de facteurs que sont le pH et la température. Quand la température est importante et est apte de participer à la destruction de cellules de virus, le pH alcalin est un peu réduit (moins agressif), tandis que lorsque la température est réduite, le pH devient plus alcalin (plus agressif). Ceci assure ainsi un traitement antiviral par l'hydroxyde de calcium dissous avec une efficacité assez constante pour une température de solution variant entre 0 et 60°C.

Il est ainsi possible de garantir une efficacité de traitement quelque soit la température de la solution, avant que la solution appliquée sur les muqueuses nasales ne prenne la température du corps humain. A la température du corps humain, le pH de la solution est d'environ 12.

La valeur du pH, bien que déjà auto régulée par l'effet tampon de l'hydroxyde de calcium dissous, peut encore être ajustée par le contrôle de la concentration en en hydroxyde de calcium dissous dans l'eau (par exemple en contrôlant la température de la solution lors de l'étape de filtration) et/ou par l'ajout d'une huile végétale sous forme de liniment.

La solution d'hydroxyde de calcium peut aisément être fabriquée dans des unités pharmaceutiques, dans des pharmacies, mais également dans des installations non de niveau pharmaceutique (par exemple dans des installations pour cosmétiques), puisque l'hydroxyde de calcium joue un rôle de désinfectant pour l'eau utilisée, pour le contenant (spray nasal, etc), et pour le milieu de travail.

Lors de nombreuses missions en Afrique dans le cadre de l'aide de l'Union Européenne aux pays en voie de développement, le demandeur a proposé un projet de potabilisation d'eau par un traitement à l'hydroxyde de calcium, dans des régions dépourvues d'hypochlorite de sodium. Les tests réalisés en Afrique ont donné des résultats exceptionnels. En effet , par un simple ajout d'une faible quantité d'hydroxyde de calcium à de l'eau portée à 35°C (eau contenue dans des cuves métalliques soumises aux rayons du soleil ), l'eau présentait un pH de 11,2 - 11,5. Par rapport à la quantité de germes pathogènes présents dans l'eau de départ, 24 heures après l'ajout d'hydroxyde de calcium, un taux de destruction des germes pathogènes de plus de 99,997% était atteint, la quantité de germes encore présents était proche des limites de détection. L'action combinée du pH et de la température a démontré l'efficacité de ces facteurs contre les germes pathogènes présents dans l'eau. Ceci conforte l'idée que la composition de l'invention peut être préparée dans des laboratoires de niveau non pharmaceutique, puisque l'hydroxyde de calcium dissous et la température sont des facteurs suffisants pour transformer l'eau de départ impure, après filtration, en une eau de qualité sanitaire propre à être bue.

Un des objets de la présente description est de mettre en exergue l'action biocide de la solution d'hydroxyde de calcium dissous (et donc de son pH très alcalin) contre les micro-organismes pathogènes, virus et bactéries. Ainsi un simple lavage des mains au moyen d'une composition à base de la solution d'hydroxyde de calcium (par exemple sous forme de savons ou de lotions) permet d'éliminer les virus et bactéries présents sur les mains d'un ouvrier en moins de 5 minutes. Cette propriété est utile pour les ouvriers employés pour le conditionnement de la solution aqueuse dans des sprays nasaux. En effet ces ouvriers n'ont qu'à prélever un peu de composition aqueuse à conditionner pour désinfecter leurs mains avant de procéder à des étapes de conditionnement de la solution.

Pasteur avait démontré que les virus et bactéries étaient rapidement éliminés en chauffant le milieu à une température de plus de 50°C (procédé de pasteurisation). Des tests ont démontré qu'une solution d'hydroxyde de calcium avec un pH de 11,5 à 12 (solution riche en ions hydroxyles) présente une action biocide identique, voire meilleure qu'un simple traitement thermique à 50°C.

Une deuxième propriété remarquable et unique de l'hydroxyde de calcium est son affinité pour les sucres. Cette propriété spécifique de la chaux a été à la base de l'essor de l'industrie sucrière à partir de la betterave. En privant les cellules des virus d'avoir accès à ces sucres suite au traitement à la chaux hydratée, les virus sont privés d'énergie et ne sont alors pas aptes pour élaborer des protéines ARN ou ADN, les empêchant ainsi de se reproduire. L'hydroxyde de calcium intervient donc en tant qu'agent antiviral, inhibant de plus la reproduction de cellules virales, et inhibant ainsi la génération de mutants.

Une troisième propriété remarquable et unique de l'hydroxyde de calcium est que sa solubilité dans l'eau est inversement proportionnelle à la température de la solution. Ainsi, sa solubilité à 0°C est d'environ 0,185g / 100 cc (1,85g /litre d'eau), tandis qu'elle n'est plus que de 0,0739g/100cc (0,739g/litre d'eau) à 100°C. Cette propriété paradoxale et singulière de l'hydroxyde de calcium limite le pH de la solution à environ 12 à une température de 36 - 37°C. En préparant la solution d'hydroxyde de calcium à une température supérieure à la température d'utilisation, le préparateur a l'assurance que la teneur en hydroxyde de calcium dissous reste constante à une température inférieure à la température de préparation, cette teneur correspondant à la teneur de saturation à cette température supérieure. Cette propriété remarquable facilite la préparation de la solution par du personnel peu qualifié. En effet ce personnel doit uniquement contrôler la température de la solution au moment de sa préparation et de sa filtration, pour obtenir un soluté avec la teneur désirée d'hydroxyde de calcium dissous.

Lors du refroidissement de la température du soluté, aucun dépôt solide ne se formera et la teneur en hydroxyde de calcium dissous restera constante.

La solution d'hydroxyde de calcium dissous assure une innocuité vis à vis des cellules ADN humaines au niveau des voies respiratoires, et une grande agressivité vis-à-vis des cellules ARN ou protéines ARN de virus. Ces propriétés sont confirmées par l'observation de pneumologues et médecins du travail de l'absence d'effets néfastes sur la santé des travailleurs de l'industrie de la chaux, et ce même en cas d'inhalation de quantité de poussières importante à une époque où les normes environnementales de particules dans l'air étaient moins sévères ou inexistantes.

Une quatrième propriété remarquable de l'hydroxyde de calcium est sa potentialité de réagir avec du gaz carbonique issu de la respiration. Ceci permet d'éliminer l'excès éventuel de chaux hydratée sous forme de carbonate. Cette propriété contribue à l'innocuité de la molécule en solution ou sous forme de micelles quelques soient les quantités absorbées ou administrées.

Si présent sur les muqueuses nasales, l'hydroxyde de calcium réagissant avec le CO₂ peut former une croûte partielle sur les muqueuses nasales, formant ainsi une barrière de protection. Cette barrière de protection est alors facilement expulsée des muqueuses, par exemple lorsque le patient se mouche.

A titre préventif, le traitement consiste à inhaler par voie nasale une solution d'hydroxyde de calcium ou à titre curatif au moyen d'un nébuliseur ou atomiseur, avantageusement électrique, qui injecte dans les voies respiratoires ou dans un masque recouvrant au moins le nez et la bouche, la solution sous forme de fines gouttelettes. Cette nébulisation ou atomisation est avantageusement réalisée à une température contrôlée, par exemple à une température de 40 à 60°C.

L'efficacité du traitement s'appuie sur les quatre propriétés de la molécule d'hydroxyde de calcium. Les effets des propriétés se cumulent, s'additionnent et combinent leurs avantages respectifs, assurant ainsi une efficacité antivirale exceptionnelle pour l'hydroxyde de calcium dissous, même si la solution aqueuse ne contient qu'une faible quantité d'hydroxyde de calcium dissous.

L'efficacité de la composition réside essentiellement sur l'action spécifique du pH (ou des ions hydroxyles) à une valeur supérieure à 11,5 à une température de 20 à 50°C vis à vis des virus à caractère acide. On est en présence d'une réaction acido-basique dont l'action létale peut être comparée à l'effet de la température qui détruit les germes pathogènes (pasteurisation). Cette action biocide est renforcée et cumulée par la propriété unique et spécifique de l'affinité de la molécule d'hydroxyde de calcium pour les sucres ( glucose, saccharose, fructose ) qui constituent la source et la réserve d'énergie pour les virus. Cette hypothèse est confortée par le fait que les glucides (C₆H₁₂O₆) très répandus dans la nature sont la source essentielle d'énergie pour les organismes vivants.

L'association des quatre propriétés de la molécule d'hydroxyde de calcium permet d'attaquer le virus par des voies distinctes, permettant une synergie d'actions antivirales, assurant une destruction rapide des virus, sans leur laisser la possibilité de muter.

L'hydroxyde de calcium dissous dans l'eau assure un effet tampon à une valeur optimale de pH assurant une innocuité aux cellules ADN de l'homme, mais une action biocide aux cellules ARN externes. L'hydroxyde de calcium permet une neutralisation des sucres empêchant ainsi les virus à avoir accès à l'énergie nécessaire à leur développement. Une fois appliquée, l'éventuel excès de chaux peut être éliminer sous forme de carbonate de calcium par l'émission d'anhydride carbonique de la respiration. Si des virus ou protéines virales sont présents dans les gaz expulsés de la respiration, ces virus ou protéines virales après être traités vont être piégés ou emprisonnés, assurant ainsi une sorte d'inertage de particules virales.

Une particularité tout à fait remarquable de ce traitement biochimique est d'atteindre des propriétés pérennes des coronavirus, et ce même en cas de mutations, de nouveaux variants et même pour de nouveaux virus à venir.

L'invention peut ainsi se résumer comme suit:
La composition de l'invention est une composition liquide de traitement antiviral et/ou antibactérien et/ou anti inflammatoire et/ou décongestionnant, en particulier un traitement combinant une activité antivirale et une activité antibactérienne, au moins au niveau de la muqueuse nasale d'un mammifère et/ou pour booster la défense immunitaire d'un mammifère contre des virus et bactéries, ladite composition liquide se présentant sous la forme d'une solution aqueuse adaptée pour une mise en contact avec la muqueuse nasale, ladite solution aqueuse liquide étant constituée à plus de 99,5% en poids d'eau déminéralisée et d'hydroxyde de calcium et comprenant de l'hydroxyde de calcium dissous, la teneur en hydroxyde de calcium dissous étant comprise entre 0,3g/litre et 3g/litre, le pH de la solution étant supérieur à 11,5 à 20°C, ladite composition étant avantageusement exempte de particules solides d'hydroxyde de calcium, de préférence de toutes particules solides. La solution aqueuse présente une tension superficielle à 20°C supérieure à 0,06 N.m⁻¹ (60 dynes/cm), de préférence égale à ou supérieure à environ 0,07 N.m⁻¹ (70 dynes/cm), par exemple mais de préférence avec une tension superficielle comprise entre 70 dynes/cm et 80 dynes/cm (1 dyne/cm = 1 milli Newton/m). La solution aqueuse a de préférence une tension superficielle proche de celle de l'eau. Des solutions avec de telles tensions superficielles se sont montrées adéquates pour assurer un dépôt rapide de plaquettes fines de chaux hydratée sur les parois ou les poils des fosses nasales.

La composition selon l'invention présente avantageusement une ou plusieurs des caractéristiques suivantes :
- la composition comprend au moins un polyéthylène glycol (PEG) de poids moléculaire inférieur à 20000, avantageusement compris entre 400 et 15000, par exemple un poids moléculaire moyen en poids de 400, 500, 800, 1000, 1200, 1500, 2000, 5000, 8000, 10000, 12000 et 15000. Des poids moléculaires moyens en poids intermédiaires sont possibles. La présence de ce polyéthylène glycol s'est avérée intéressante car assurant un meilleur pompage de la solution et donc un meilleur dosage de la pompe du spray et une absence de formation de bouchage au niveau de l'ouverture ou des ouvertures de l'embout de pulvérisation.
- la composition comprend du NaCl dissous dans la solution à raison de moins de 3g/litre, avantageusement de manière à ce que le rapport en poids hydroxyde de calcium dissous / NaCl dissous soit compris entre 0,1 et 2, de préférence entre 0,2 et 1, en particulier entre 0,3 et 0,7, et/ou
   du Na₂CO₃ dissous dans la solution à raison de moins de 3g/litre, avantageusement de manière à ce que le rapport en poids hydroxyde de calcium dissous / Na₂CO₃ dissous soit compris entre 0,1 et 2, de préférence entre 0,2 et 1, en particulier entre 0,3 et 0,7, et/ou
   du NaHCO₃ dissous dans la solution à raison de moins de 3g/litre, avantageusement de manière à ce que le rapport en poids hydroxyde de calcium dissous / NaHCO₃ dissous soit compris entre 0,1 et 2, de préférence entre 0,2 et 1, en particulier entre 0,3 et 0,7.
   A titre d'exemples de rapport en poids, on peut citer 0,1 ; 0,2 ; 0,3 ; 0,4 ; 0,5 ; 0,6 ; 0,7 ; 0,8 et 0,9. La présence d'une faible quantité de sel de sodium (Chlorure, carbonate ou bicarbonate) dissous dans la solution permet de booster l'effet de la chaux hydratée dans les narines. Sans être lié à une quelconque théorie, il semble que la présence de sel de sodium dissous (NaCl, Na₂CO₃ ou NaHCO₃) permet de concentrer le dépôt de chaux hydratée sous forme de film ou micro plaquettes au niveau des narines.
- la composition comprend du NaCl et/ou du carbonate de sodium et/ou du bicarbonate de sodium en tant que sel(s) dissous dans la solution, ledit sel ou lesdits sels dissous dans la solution étant présent(s) à raison de moins de 3g/l , avantageusement de manière à ce que le rapport en poids hydroxyde de calcium dissous dans la solution / NaCl et/ou carbonate de sodium et/ou bicarbonate de sodium dissous dans la solution soit compris entre 0,1 et 2, de préférence entre 0,2 et 1, en particulier entre 0,3 et 0,7.
- la teneur en hydroxyde de calcium et/ou la teneur en polyéthylène glycol de poids moléculaire inférieur à 20000 et/ou la teneur en NaCl et/ou la teneur en carbonate de sodium et/ou la teneur en bicarbonate de sodium dans la solution est/sont adaptées pour que la viscosité dynamique de la solution à 20°C soit comprise entre 0,8 x 10⁻³ Pa.s et 1,2 x 10⁻³ Pa.s.
- la composition comprend une huile végétale, sous forme de micro ou nano émulsion, cette huile végétale formant de préférence un liniment.
- la composition est destinée pour application à une température de moins de 40°C sur au moins une muqueuse nasale d'un mammifère. La composition est caractérisée en ce que la composition comprenant de l'hydroxyde de calcium dissous a été soumise à une étape de chauffage à une température comprise entre 60 et 95°C, et à une étape de séparation de la solution d'hydroxyde de calcium à une température comprise entre 60 et 95°C. Si la composition comprend d'autres composés (tels que par exemple du NaCl, du Polyéthylène glycol, etc.), la composition est chauffée à une température comprise entre 60 et 95°C, avant d'être filtrée à cette température. Avantageusement, la composition est maintenue à une température de 60 à 95°C pendant au moins 3 minutes (par exemple entre 5 et 10 minutes) avant d'être filtrée.
- la composition est préparée à partir d'hydroxyde de calcium ou d'oxyde de calcium présentant une pureté de plus de 99,0%, avantageusement de plus de 99,5%, en particulier de plus de 99,9%.
- la composition est constituée à plus de 99% en poids, avantageusement à plus de 99,5% en poids, de préférence à plus de 99,9% en poids d'eau déminéralisée et d'hydroxyde de calcium.

La description décrit aussi un dispositif nasal ou dispositif d'administration nasale comprenant une composition suivant l'invention, ledit dispositif se présentant sous la forme d'un spray nasal ou d'un inhalateur nasal (par exemple un nébuliseur, par exemple sous pression ou avec un mécanisme de mise sous pression) ou d'un puff nasal.

Avantageusement, le dispositif nasal est adapté pour l'administration de doses unitaires de composition comprises entre 25µl et 200µl, en particulier de 50µl à 100µl. Le dispositif nasal comprend un moyen d'administration de doses unitaires, tel que par exemple une pompe bouton de pulvérisation, ladite pompe étant avantageusement actionnable de manière manuelle. Le dispositif nasal présente avantageusement un embout adapté pour rentrer dans une fosse nasale avant l'activation de la pompe ou micropompe.

L'invention a encore pour objet un procédé de préparation d'une composition selon l'invention, dans lequel la solution d'hydroxyde de calcium est préparée à une température comprise entre 60°C et 95°C, avantageusement entre 60°C et 80°C, cette solution étant filtrée à une température comprise entre 60°C et 95°C, avantageusement entre 60 et 80°C, avant d'être conditionnée avantageusement à chaud dans un dispositif d'administration ou dans une recharge pour dispositif d'administration (par exemple un spray nasal ou inhalateur avec une micropompe).

La production industrielle (dans des unités industrielles ou dans des laboratoires ou dans des pharmacies) de solutions ou de micelles d'hydroxyde de calcium est avantageusement réalisée par ajustement de la concentration en hydroxyde de calcium dissous des solutés en fonction de la température de sa préparation. Sa solubilité réduite à haute température permet d'empêcher une obturation des conduits d'injection ou de pulvérisation du spray nasal ou atomiseur (par exemple électrique) à sa température d'utilisation. Lorsque la solution est préparée à une température de saturation supérieure à 50°C, voire plus, la solution peut être maintenue et conservée à une température ambiante ou au frigo sans risque de dépôt dans le dispositif d'administration ou dans sa recharge, sans risque de variation de sa teneur en ions hydroxyles au cour du temps.

Dans le cadre de l'invention, la solution d'hydroxyde de calcium peut être combinée à une huile végétale pour former un liniment en vue de réduire le pH ou de stabiliser son pH à une valeur proche de 11,5, voire légèrement en dessous de 11,5.

Pour la préparation des solutions de chaux hydratée, on utilisera avantageusement de l'hydroxyde de calcium en poudre présentant une granulométrie moyenne en poids de moins de 50µm, en particulier de moins de 20µm, et présentant une grande pureté (la chaux hydratée étant constituée à plus de 99,5% en poids, en particulier à plus de 99,9% en poids de Ca(OH)₂ et d'eau) et présentant une grande surface spécifique (avantageusement de plus de 40m²/g).

Les exemples suivants montrent l'innocuité, l'efficacité et la simplicité des traitements selon l'invention.

### Exemple 1

Pour ce test sur des volontaires (personnes présentant un âge de plus de 60 ans), on a préparé une solution de chaux hydratée à la température de 60°C, cette solution contenant 1g de Ca(OH)₂ dissous. Le soluté a été filtré à 60°C et a été conditionné dans des flacons de 50ml . Les flacons ont été fermés au moyen de têtes de pulvérisation adaptées pour l'injection de doses unitaires de 100µl par opération de pompage. La tension superficielle à 20°C était supérieure à 70 dynes/cm ou 70 milliNewton/m. La viscosité de la solution à 20°C était d'environ 1 10⁻³ Pa.s

Le traitement des volontaires consistait à injecter, trois fois par jour dans chacune de leurs narines, deux doses unitaires de 100µl de solution d'hydroxyde de calcium. Ce traitement a été effectué sur une période de 16 mois. Tous les mois, les volontaires recevaient de nouveaux flacons.

Le contrôle de la prise régulière de la solution à injecter dans les narines est effectué quotidiennement par mesure du poids des flacons. Ceci permet de vérifier la posologie journalière et le bon suivi du traitement par les volontaires.

Durant cette période de 16 mois de la pandémie COVID-19, il a été remarqué ce qui suit:
- les volontaires ont adhéré au traitement, de par sa simplicité, l'absence d'effets secondaires (innocuité), et un ressenti d'un état de santé amélioré;
- bien que vaccinés, les volontaires n'ont pas développé de symptômes caractéristiques d'une infection au Covid-19, alors que pour un groupe témoin (vacciné), les personnes ont développé des symptômes liés à une infection à la Covid-19, avec pour certains une période d'hospitalisation (réanimation, conduisant pour certains à un décès). Ceci démontre une efficacité du traitement selon l'invention contre la Covid-19, contre sa propagation, contre une contamination à la Covid-19 et/ou à ses variants.

### Exemple 2

Des volontaires d'une maison de repos (plus de 75 ans, vaccinés, non traités en préventif au moyen de la composition selon l'invention) qui ont manifesté des premiers symptômes caractéristiques d'une infection à la Covid-19 (fièvre, toux, perte de goût, fatigue et courbatures) ont été immédiatement traités au moyen d'un masque relié à un atomiseur électrique atomisant à 30°C en 15 minutes 25 ml de solution d'hydroxyde de calcium. Ce traitement a été opéré trois fois par jour.

Les volontaires ont adhéré au traitement, vu l'absence total d'effets secondaires, et vu un ressenti d'une amélioration rapide dès après un traitement. Après une semaine de traitement, les volontaires ne présentaient plus de symptômes liés à une infection à la Covid-19.

### Exemple 3

La composition de l'exemple 1 a été modifiée de la manière suivante pour préparer les compositions suivantes.

exemple 3A : A la composition utilisée à l'exemple 1 (encore chaude (plus de 60°C), et avant filtration), on a ajouté 0,5g/litre de NaCl. Après mélange et filtration, la solution avait une tension superficielle à 20°C légèrement supérieure à celle de la composition utilisée à l'exemple 1.

exemple 3B : A la composition utilisée à l'exemple 1 (encore chaude et avant sa filtration à 70°C), on a ajouté 0,5g/litre de polyéthylène glycol de poids moléculaire moyen en poids de 1000. Après mélange avec maintien de la solution à une température de 70°C pendant 5 minutes, et filtration, la solution avait une tension superficielle à 20°C de l'ordre de 0,65 dynes/cm.

exemple 3C : A la composition utilisée à l'exemple 1 (encore chaude et avant sa filtration à 70°C), on a ajouté 1g/l de NaCl et 0,5g/litre de polyéthylène glycol de poids moléculaire moyen en poids de 1000. Après mélange avec maintien de la solution à une température de 70°C pendant 5 minutes, et filtration, la solution avait une tension superficielle à 20°C de l'ordre de 0,70 dynes/cm.

exemple 3D : A la composition utilisée à l'exemple 1 (encore chaude (plus de 60°C), et avant filtration), on a ajouté 0,5g/litre de NaHCO₃. Après mélange et filtration, la solution avait une tension superficielle à 20°C légèrement supérieure à celle de la composition utilisée à l'exemple 1.

exemple 3E : A la composition utilisée à l'exemple 1 (encore chaude (plus de 60°C), et avant filtration), on a ajouté 0,5g/litre de NaCl et 0,5g/l de NaHCO₃. Après mélange et filtration, la solution avait une tension superficielle à 20°C légèrement supérieure à celle de la composition utilisée à l'exemple 1.

## Revendications

1. Composition liquide de traitement antiviral et/ou antibactérien et/ou anti inflammatoire et/ou décongestionnant au moins au niveau de la muqueuse nasale d'un mammifère et/ou pour booster la défense immunitaire d'un mammifère, ladite composition liquide se présentant sous la forme d'une solution aqueuse à mettre en contact avec la muqueuse nasale, ladite solution aqueuse liquide étant constituée à plus de 99,5% en poids d'eau déminéralisée et d'hydroxyde de calcium et comprenant de l'hydroxyde de calcium dissous avec une teneur en hydroxyde de calcium dissous comprise entre 0,3g/litre et 3g/litre, le pH de la solution étant supérieur à 11,5 à 20°C, tandis que la solution aqueuse présente une tension superficielle à 20°C supérieure à 0,06 N.m-1 (60 dynes/cm), de préférence égale ou supérieure à environ 0,07 N.m-1 (70 dynes/cm), ladite composition étant exempte de particules solides d'hydroxyde de calcium, et autres particules solides.

2. Composition selon la revendication 1, **caractérisée en ce que** la composition comprend au moins un polyéthylène glycol de poids moléculaire inférieur à 20000.

3. Composition suivant la revendication 1 ou 2, **caractérisée en ce qu'**elle comprend du NaCl et/ou du carbonate de sodium et/ou du bicarbonate de sodium en tant que sel(s) de sodium dissous dans la solution , ledit sel ou lesdits sels de sodium dissous étant présent(s) dans la solution à moins de 3g/l.

4. Composition suivant l'une quelconque des revendications précédentes, **caractérisée en ce que** la teneur en hydroxyde de calcium et/ou la teneur en polyéthylène glycol et/ou la teneur en NaCl et/ou la teneur en Na₂CO₃ et/ou la teneur en NaHCO₃ dans la solution est/sont adaptées pour que la viscosité dynamique de la solution à 20°C est comprise entre 0,8 × 10⁻³ Pa.s et 1,2 × 10⁻³ Pa.s.

5. Composition selon l'une quelconque des revendications précédentes, **caractérisée en ce que** la composition comprend une huile végétale, sous forme de micro ou nano émulsion.

6. Composition suivant l'une des revendications précédentes pour application à une température de moins de 40°C sur une muqueuse nasale d'un mammifère, **caractérisée en ce que** la composition comprenant de l'hydroxyde de calcium dissous a été soumise à une étape de chauffage à une température comprise entre 60 et 95°C, et à une étape de séparation de la solution d'hydroxyde de calcium à une température comprise entre 60 et 95°C.

7. Composition selon l'une quelconque des revendications précédentes, **caractérisée en ce qu'**elle est préparée à partir d'hydroxyde de calcium en poudre présentant avantageusement une surface spécifique BET de plus de 25m² /g ou d'oxyde de calcium en poudre, ledit hydroxyde de calcium en poudre ou ledit oxyde de calcium en poudre présentant avantageusement une pureté de plus de 99,0%, avantageusement de plus de 99,5%, en particulier de plus de 99,9%.

8. Composition suivant l'une quelconque des revendications précédentes, **caractérisée en ce qu'**elle est constituée à plus de 99,9% en poids d'eau déminéralisée et d'hydroxyde de calcium.

9. Dispositif d'administration nasale comprenant une composition suivant l'une des revendications précédentes, ledit dispositif se présentant sous la forme d'un spray nasal ou d'un inhalateur nasal ou puff nasal.

10. Dispositif d'administration nasale selon la revendication précédente, **caractérisé en ce qu'**il est adapté pour l'administration de doses unitaires de composition comprises entre 25µl et 200µl, en particulier de 50µl à 100µl.

11. Procédé de préparation d'une composition selon l'une quelconque des revendications 1 à 8, **caractérisé en ce que** la solution d'hydroxyde de calcium est préparée à une température comprise entre 60°C et 95°C, avantageusement entre 60°C et 80°C, cette solution étant filtrée à une température comprise entre 60 et 95°C, de préférence entre 60°C et 80°C, avant d'être conditionnée avantageusement à chaud dans un dispositif d'administration nasale ou dans une recharge pour un dispositif d'administration nasale.

## Patentansprüche

1. Flüssige Zusammensetzung zur antiviralen und/oder antibakteriellen und/oder antiinflammatorischen und/oder dekongestierenden Behandlung mindestens an der Nasenschleimhaut eines Säugers und/oder zur Verstärkung der Immunabwehr eines Säugetiers, wobei die flüssige Zusammensetzung in Form einer wässrigen Lösung vorliegt, die mit der Nasenschleimhaut in Kontakt gebracht werden soll, wobei die flüssige wässrige Lösung zu mehr als 99,5 Gew.-% aus demineralisiertem Wasser und gelöste Calciumhydroxiden besteht. wobei der Gehalt an gelöstem Calciumhydroxid zwischen 0,3 g/liter und 3g/liter liegt, wobei der pH-Wert der Lösung bei 20° C größer als 11,5 ist, während die wässrige Lösung eine Oberflächenspannung bei 20° C von mehr als 0,06 N.m⁻¹ ( 60 dynes/cm), vorzugsweise gleich oder grösser als etwa 0,07 N.m⁻¹ (70 dynes/cm), aufweist, wobei die Zusammensetzung frei von festen Partikeln von Calciumhydroxiden ist, und frei von anderen Feststoffpartikeln.

2. Zusammensetzung nach Anspruch 1, **dadurch gekennzeichnet, dass** die Zusammensetzung mindestens ein Polyethylenglykol mit einem Molekulargewicht von weniger als 20000 umfaßt.

3. Zusammensetzung nach Anspruch 1 oder 2, **dadurch gekennzeichnet, dass** sie NaCl und/oder Natriumcarbonat und/oder Natriumbicarbonat als in dem Lösungsmittel gelöste Salz (e) umfaßt, wobei das oder die in der Lösung gelösten Salze in einem Anteil von weniger als 3 g/l.

4. Zusammensetzung nach einem der vorhergehenden Ansprüche, **dadurch gekennzeichnet, dass** der Gehalt an Calciumhydroxid und/oder der Gehalt an Polyethylenglykol und/oder der Gehalt an NaCl und/oder der Gehalt an Na₂CO₃ und/oder der Gehalt an NaHCO₃ in der Lösung so angepasst sind, dass die dynamische Viskosität der Lösung bei 20° C zwischen 0,8 × 10⁻³ Pa.s und 1,2 × 10⁻³ Pa.s liegt.

5. Zusammensetzung nach einem der vorhergehenden Ansprüche, **dadurch gekennzeichnet, dass** die Zusammensetzung ein Pflanzenöl in Form von Mikro- oder Nanoemulsionierung umfaßt.

6. Zusammensetzung nach einem der vorhergehenden Ansprüche zur Anwendung bei einer Temperatur von weniger als 40° C auf einer Nasenschleimhaut eines Säugetiers, **dadurch gekennzeichnet, dass** die Zusammensetzung, die gelöstes Calciumhydroxid umfaßt, einem Erwärmungsschritt bei einer Temperatur zwischen 60 und 95° C und einem Schritt des Trennens der Calciumhydroxidlösung bei einer Temperatur zwischen 60 und 95° C unterzogen wurde.

7. Zusammensetzung nach einem der vorhergehenden Ansprüche, **dadurch gekennzeichnet, dass** sie aus pulverförmigem Calciumhydroxid hergestellt ist, das vorteilhafterweise eine spezifische BET-Oberfläche von mehr als 25 m²/g oder von pulverförmigem Calciumoxyde, aufweist, wobei das pulverförmige Calciumhydroxid oder das pulverförmige Calciumoxid vorteilhafterweise eine Reinheit von mehr als 99,0 %, vorteilhafterweise von mehr als 99,5 %, insbesondere von mehr als 99,9 %°, aufweist.

8. Zusammensetzung nach einem der vorhergehenden Ansprüche, **dadurch gekennzeichnet, dass** sie zu mehr als 99,9 Gew.-% aus demineralisiertem Wasser und Calciumhydroxiden besteht.

9. Nasale Abgabevorrichtung, umfassend eine Zusammensetzung nach einem der vorhergehenden Ansprüche, wobei die Vorrichtung in Form eines Nasensprays oder nasalen oder nasalen Zuginhalators vorliegt.

10. Nasale Abgabevorrichtung nach dem vorhergehenden Anspruch, **dadurch gekennzeichnet, dass** sie für die Verabreichung von Einheitsdosen von Zusammensetzung, die zwischen 25 µl und 200 µl, insbesondere von 50 µl bis 100 µm umfaßt, angepasst ist.

11. Verfahren zur Herstellung einer Zusammensetzung nach einem der Ansprüche 1 bis 8, **dadurch gekennzeichnet, dass** die Calciumhydroxidlösung bei einer Temperatur zwischen 60° C und 95° C, vorteilhafterweise zwischen 60° C und 80° C, hergestellt wird, wobei diese Lösung bei einer Temperatur zwischen 60 und 95° C, vorzugsweise zwischen 60° C und 80° C, gefiltert wird, bevor sie vorteilhaft heiss in einer nasalen Abgabevorrichtung oder in einer Nachfüllung für eine nasale Abgabevorrichtung konditioniert wird.

## Claims

1. A liquid composition for antiviral and/or antibacterial and/or antiinflammatory and/or decongestant treatment at least at the nasal mucosa of a mammal and/or for boosting the immune defence of a mammal, said liquid composition being in the form of an aqueous solution to be contacted with the nasal mucosa, said liquid aqueous solution consisting of more than 99.5% by weight of demineralized water and dissolved calcium hydroxide with a dissolved calcium hydroxide content comprised between 0.3 g/liter and 3g/liter, the pH of the solution being greater than 11.5 at 20° C, while the aqueous solution has a surface tension at 20°C greater than 0.06 N.m⁻¹ (60 dynes/cm), preferably equal to or greater than about 0.07 N.m⁻¹ (70 dynes/cm), said composition being free of solid particles of calcium hydroxide, and other solid particles.

2. Composition according to claim 1, **characterized in that** the composition comprises at least one polyethylene glycol with a molecular weight of less than 20000.

3. Composition according to claim 1 or 2, **characterized in that** it comprises NaCl and/or sodium carbonate and/or sodium bicarbonate as sodium salt(s) dissolved in the solution, the said sodium salt or salts being present in the solution in a proportion of less than 3g/liter.

4. Composition according to any one of the preceding claims, **characterized in that** the content of calcium hydroxide and/or the content of polyethylene glycol and/or the content of NaCl and/or the content of Na₂CO₃ and/or the content of NaHCO₃ in the solution is/are adapted so that the dynamic viscosity of the solution at 20°C is comprised between 0.8 × 10⁻³ Pa.s and 1.2 × 10⁻³ Pa.s

5. Composition according to any one of the preceding claims, **characterized in that** the composition comprises a vegetable oil, in the form of micro-or nano-emulsion.

6. Composition according to one of the preceding claims for application at a temperature of less than 40°C on a nasal mucosa of a mammal, **characterized in that** the composition comprising dissolved calcium hydroxide has been subjected to a heating step at a temperature of between 60 and 95° C, and to a step of separating the calcium hydroxide solution at a temperature of between 60 and 95° C.

7. Composition according to any one of the preceding claims, **characterized in that** it is prepared from powdered calcium hydroxide advantageously having a BET specific surface area of more than 25m²/g or of powdered calcium oxide, said powdered calcium hydroxide or powdered calcium oxide advantageously having a purity of more than 99.0%, advantageously of more than 99.5%, in particular of more than 99.9%.

8. Composition according to any one of the preceding claims, **characterized in that** it consists of more than 99.9% by weight of demineralized water and calcium hydroxide.

9. Nasal delivery device comprising a composition according to one of the preceding claims, wherein said device is in the form of a nasal spray or nasal or nasal puff inhaler.

10. Nasal delivery device according to the preceding claim, **characterized in that** it is adapted for the administration of unit doses of composition comprised between 25 µl and 200 µl, in particular from 50 µl to 100 µl.

11. Process for the preparation of a composition according to any one of claims 1 to 8, **characterized in that** the calcium hydroxide solution is prepared at a temperature comprised between 60°C and 95° C, advantageously between 60°C and 80° C, this solution being filtered at a temperature comprised between 60 and 95° C, preferably between 60°C and 80° C, before being conditioned advantageously hot in a nasal delivery device or in a refill for a nasal delivery device.
